Europäisches Patentamt

European Patent Office

Office européen des brevets

(11) Veröffentlichungsnummer: **0 306 778 B1**

# EUROPÄISCHE PATENTSCHRIFT

(45) Veröffentlichungstag der Patentschrift: **11.03.92**

(21) Anmeldenummer: **88113902.6**

(22) Anmeldetag: **26.08.88**

(51) Int. Cl.⁵: **A61K 41/00**, A61K 31/66,
//(A61K31/66,31:365,31:575)

(54) Verfahren zur Sterilisation von Plasma oder Plasmafraktionen.

(30) Priorität: **11.09.87 DE 3730533**

(43) Veröffentlichungstag der Anmeldung:
**15.03.89 Patentblatt 89/11**

(45) Bekanntmachung des Hinweises auf die
Patenterteilung:
**11.03.92 Patentblatt 92/11**

(84) Benannte Vertragsstaaten:
**AT BE CH DE ES FR GB GR IT LI LU NL SE**

(56) Entgegenhaltungen:

**DEVELOP. BIOL. STANDARD, Band 54, 1983,
Seiten 491-495, Basel, CH; W. STEPHAN et
al.: "Factor VIII concentrate from cold sterili-
zed human plasma"**

**CHEMICAL ABSTRACTS, Band 103, Nr. 17, 28.
Oktober 1985, Seite 413, Zusammenfassung
Nr. 138446b, Columbus, Ohio, US; A.M. PRIN-
CE et al.: "Ouantitative assays for evaluation
of HTLV-III inactivation procedures:
Tri(N-butyl)phosphate:sodium cholate and
beta-propiolactone", & CANCER RES. 1985,
45(9, Suppl.), 4592-4**

(73) Patentinhaber: **BIOTEST PHARMA GMBH
Landsteiner Strasse 5
W-6072 Dreieich(DE)**

(72) Erfinder: **Dichtelmüller, Herbert, Dr.
Rossertstrasse 14
W-6231 Sulzbach/Ts(DE)**
Erfinder: **Möller, Wolfgang, Dr.
Graf-von-Stauffenberg-Strasse 32
W-6370 Oberursel(DE)**
Erfinder: **Stephan, Wolfgang, Dr.
Philipp-Holzmann-Strasse 84
W-6072 Dreieich(DE)**
Erfinder: **Schleussner, Hans, Dr.
Nansenring 26
W-6000 Frankturt/Main 70(DE)**

(74) Vertreter: **Wolff, Hans Joachim, Dr.jur.
Dipl.-Chem. et al
Beil, Wolff & Beil Rechtsanwälte Postfach 80
01 40 Adelonstrasse 58
W-6230 Frankturt am Main 80(DE)**

**Beschreibung**

Die Erfindung betrifft das in den Patentansprüchen beschriebene Verfahren zur Sterilisation von Plasma oder Plasmafraktionen einschließlich Präparaten, die den Blutgerinnungsfaktor VIII enthalten.

Humanes Plasma oder daraus hergestellte Plasmaderivate enthalten potentiell humanpathogene Viren, die den Empfänger dieser Präparate dem Risiko einer Infektion mit Hepatitis B Virus, Hepatitis Non-A, Non-B (NANB) Virus, oder HIV (AIDS) aussetzen. Humane Plasmapräparate müssen deshalb einem wirksamen Sterilisationsverfahren unterzogen werden, z. B. durch Hitzebehandlung, Behandlung mit Chemikalien oder Bestrahlung. Eine Hitzebehandlung ist für Präparate, die Gerinnungsfaktoren enthalten, nicht geeignet, da die Gerinnungsfaktoren schon durch einstündiges Erhitzen auf 60°C zerstört werden.

Von LoGrippo wurde ein Sterilisationsverfahren vorgeschlagen (vgl. G.A. LoGrippo u.a., "Chemical and Combined Methods for Plasma Sterilization", Bibl. Haematol., Bd. 7 (1958), S. 225 bis 230), bei dem Humanplasma mit $\beta$-Propiolacton behandelt wurde und diese Behandlung mit einer Bestrahlung mit UV-Strahlen kombiniert wurde. Durch dieses Verfahren werden die relevanten humanpathogenen Viren, HBV, HNANBV, HIV mit hoher Effektivität inaktiviert, ebenso Bakteriophagen, Sendai-Virus und zahlreiche andere Viren, unabhängig von Virusgröße, Genom oder Hüllstruktur. Es werden sowohl RNA- als auch DNA-Viren, Viren mit Lipidhülle ("Lipidviren") oder Proteinhüllen ("Proteinviren") gleichermaßen inaktiviert. Die Effektivität des $\beta$-Propiolacton/UV-Verfahrens ist so hoch, daß auch sensible Plasmaproteine, insbesondere der Blutgerinnungsfaktor VIII (F VIII), an Aktivität verlieren. So ist es bisher nicht gelungen, mittels des $\beta$-Propiolacton/UV-Verfahrens Faktor VIII mit ausreichender Ausbeute zu sterilisieren, obwohl andere Plasmaproteine, wie Serumkonserven, der Prothrombinkomplex (PPSB) oder Fibrinogen erfolgreich diesem Verfahren unterzogen werden können.

$\beta$-Propiolacton reagiert mit RNA (Ribonukleinsäure) oder DNA (Desoxyribonukleinsäure), in geringerem Maße mit Proteinen und wird in einer dritten Reaktion mit Wasser zu $\beta$-Hydroxypropionsäure abgebaut.

Durch UV-Strahlung wird RNA oder DNA sowohl durch intramolekulare Reaktionen als auch durch Spaltung zerstört. Die Wirksamkeit der UV-Bestrahlung ist dabei um so höher je verdünnter die Proteinlösung und je niedriger die optische Dichte bei 254 nm ist.

Aus der DE PS 30 33 932 ist ein Verfahren zur Kaltsterilisation von Blutgerinnungsfaktor VIII enthaltenden Präparaten bekannt, bei dem die den F VIII enthaltende Proteinlösung in 4 Stufen mit einem nichtionogenen Tensid behandelt, mit UV-Licht bestrahlt, mit $\beta$-Propiolacton behandelt und einer Adsorptionsbehandlung mit kolloidaler Kieselsäure unterworfen wird. Auch bei diesem umständlichen Verfahren wurde jedoch noch keine gute Ausbeute an Faktor VIII-Aktivität erreicht.

Aus Transfusion, Bd. 25, Nr. 6 (1985), Seiten 516 bis 522, ist ein weiteres Kaltsterilisationsverfahren bekannt, welches Viren mit Lipidhülle (Lipidviren) durch Behandlung mit Tri-n-butylphosphat/Natriumcholat (TNBP/NC) inaktiviert. Für dieses Verfahren wurde zwar eine Inaktivierung von Hepatitis B Virus, Hepatitis NANB Virus und HIV (AIDS) nachgewiesen, und auch Faktor VIII ist ohne entscheidende Verluste an Aktivität durch dieses Verfahren sterilisierbar; dieses TNBP/NC-Verfahren bewirkt jedoch keine Inaktivierung von Viren, deren Hülle keine Lipidbestandteile aufweist. "Proteinviren" (z. B. Øx 174) werden nicht inaktiviert, so daß dieses Verfahren auf Lipidviren beschränkt ist.

Der Erfindung liegt die Aufgabe zugrunde, ein Verfahren zur Sterilisation von Plasma oder Plasmafraktionen einschließlich solcher Fraktionen, die den Blutgerinnungsfaktor VIII enthalten, unter Behandlung mit $\beta$-Propiolacton und/oder UV-Strahlung bereitzustellen, das einerseits eine verbesserte Ausbeute an Faktor VIII-Aktivität ergibt und andererseits sowohl die humanpathogenen Lipidviren als auch die humanpathogenen Proteinviren inaktiviert.

Diese Aufgabe wird erfindungsgemäß dadurch gelöst, daß man vor oder gleichzeitig mit der Behandlung mit $\beta$-Propiolacton oder UV-Strahlung eine Behandlung mit Tri-n-butylphosphat und Natriumcholat oder Tween® 80 durchführt.

Überraschenderweise wurde festgestellt, daß durch die erfindungsgemäße Kombination einer Behandlung mit $\beta$-Propiolacton oder UV-Strahlung mit einer Behandlung mit Tri-n-butylphosphat und Natriumcholat oder Tween® 80 eine drastische und synergistische Steigerung der Inaktivierung von Lipidviren und Proteinviren bewirkt wird, obwohl Tri-n-butylphosphat und Natriumcholat oder Tween® 80 allein gegen Proteinviren nahezu oder vollkommen unwirksam sind. Offensichtlich wird durch das Tri-n-butylphosphat und Natriumcholat oder Tween® 80 die im Plasma und in den Plasmafraktionen vorhandene, das $\beta$-Propiolacton abbauende Aktivität beseitigt und dadurch eine deutliche Steigerung der Wirksamkeit des $\beta$-Propiolactons bewirkt. Die synergistische Wirkung von UV-Strahlung mit Tri-n-butylphosphat und Natriumcholat oder Tween® 80 könnte auf einer gewissen Destabilisierung der Protein- und Lipidviren durch die Lösungsmittel/Detergens-Behandlung beruhen. Die Kombinationsmethode mit UV-Strahlung führt zu einer höheren Inaktivierung als die Summe der Einzeschritte alleine. Darüberhinaus wird durch das erfindungsgemäße kombinierte Sterilisationsverfahren die Faktor VIII-Aktivität nicht wesentlich vermindert.

Das erfindungsgemäße Sterilisationsverfahren kann mit Plasma, d. h. Humanblutplasma und Plasmafraktionen durchgeführt werden. Als Plasmafraktionen, die den Blutgerinnungsfaktor VIII enthalten, können Kryopräzipitat oder eine Cohn-Fraktion I verwendet werden. Die Sterilisation wird bei einem pH-Wert von 4,5 bis 8,3, vorzugsweise bei einem pH-Wert von 7,2 und bei einer Temperatur von +4 bis 37°C, vorzugsweise bei 23°C, durchgeführt. Die Behandlung mit Tri-n-butylphosphat und Natriumcholat oder Tween® 80 wird vor oder gleichzeitig mit der Behandlung mit $\beta$-Propiolacton oder UV-Strahlung durchgeführt. Vorzugsweise erfolgt zuerst eine Inkubierung mit Tri-n-butylphosphat und Natriumcholat oder Tween® 80 für eine Dauer von 60 bis 90 min. und dann eine Behandlung mit $\beta$-Propiolacton während weiterer 60 bis 90 min. oder eine Bestrahlung mit UV-Licht. Dabei werden das Tri-n-butylphosphat in einer Konzentration von 0,05 bis 0,5 %, vorzugsweise 0,3 %, und das Natriumcholat in einer Konzentration von 0,03 bis 0,4 %, vorzugsweise 0,2 %, angewendet. Bei Verwendung von Tween 80 anstellte von Cholat wird dieses in einer Konzentration von vorzugsweise 1 % eingesetzt. Das $\beta$-Propiolacton wird in einer Konzentration von 0,01 bis 0,3 % angewendet. Wenn eine Plasmafraktion, die den Faktor VIII enthält, sterilisiert werden soll, wird das $\beta$-Propiolacton in einer Konzentration von 0,01 bis 0,25 %, vorzugsweise 0,05 % angewendet. Der pH-Wert wird durch Zugabe von Natronlauge konstant gehalten.

Die Bestrahlung mit UV-Licht wird mit einer Intensität durchgeführt, für die eine zuverlässige Inaktivierung von Viren ausreichend ist. Beispielsweise wird die Bestrahlung in einer Rotationsdurchflußapparatur mit 2 x 20 Watt UV-Lampen bei 500-700 UPM (Umdrehungen pro Minute) und einem Durchfluß von 20 l pro Stunde bei einer Schichtdicke von 1 mm in 1 cm Abstand durchgeführt.

In den nachstehenden Beispielen wird gezeigt, daß die Aktivität von Faktor VIII durch das erfindungsgemäße kombinierte Verfahren nicht wesentlich vermindert wird und daß die Inaktivierung von Proteinviren durch die Kombination einer Behandlung mit $\beta$-Propiolacton und/oder UV-Strahlung mit einer Vorbehandlung oder gleichzeitigen Behandlung mit Tri-n-butylphosphat und Natriumcholat oder Tween® 80 gegenüber einer Inaktivierung mit $\beta$-Propiolacton und/oder UV-Strahlung allein drastisch gesteigert wird, obwohl eine Behandlung mit Tri-n-butylphosphat und Natriumcholat oder Tween® 80 allein für eine solche Inaktivierung nahezu bzw. vollkommen unwirksam ist.

Tri-n-butylphosphat/Natriumcholat bzw. Tri-n-butylphosphat/Tween® 80 dient somit hier nicht zur Inaktivierung von Viren, sondern zur Steigerung der Wirksamkeit von $\beta$-Propiolacton bzw. der UV-Bestrahlung.

Die Erfindung wird durch die folgenden Beispiele erläutert.

Beispiel 1

100 ml Humanplasma, befreit von Kryopräzipitat, wurden mit 0,3 % Tri-n-butylphosphat und 0,2 % Natriumcholat versetzt und 60 min. bei pH 7,2 und 23°C behandelt.
Anschließend wurden 50 µl $\beta$-Propiolacton (0,05 %) zugesetzt und das Plasma weitere 60 min. bei 23°C und pH 7,2 inkubiert. Gemessen wurde die F VIII-Aktivität nach $\beta$-Propiolacton Einwirkung mit und ohne Kombination mit TNBP. Von der Faktor VIII Aktivität in Plasma blieben 78 % nach $\beta$-Propiolacton-Behandlung, 58 % nach TNBP-Cholat-Behandlung und 62 % nach kombinierter Behandlung erhalten (unbehandelte Kontrolle = 100 %).

Beispiel 2

100 ml Plasma wurden wie in Beispiel 1 mit Tri-n-butylphosphat/Natriumcholat vorbehandelt und anschließend mit 0,25 % $\beta$-Propiolacton sterilisiert. Gemessen wurde die Inaktivierung des zuvor zugesetz-ten Bakteriophagen Øx 174 im Vergleich zu nicht TNBP-Cholat vorbehandeltem Plasma. Gefunden wurde eine Steigerung der Inaktivierung von Øx 174 um den Faktor ~ 72.000 im Vergleich zu nicht TNBP-Cholat vorbehandeltem, $\beta$-Propiolacton sterilisierten Plasma.

Beispiel 3

100 ml Kryopräzipitat (F VIII), gelöst in 0,15 M Glycin, wurden mit 50 µl $\beta$-Propiolacton (0,05 %) versetzt und 90 min. bei pH 7,2 und 23°C inkubiert.
Gemessen wurde die Inaktivierung des zuvor zugesetzten Bakteriophagen Øx 174. Erzielt wird eine nahezu 20fache Steigerung der Inaktivierung durch die Kombination TNBP-Cholat + $\beta$-Propiolacton im Gegensatz zur $\beta$-Propiolacton-Behandlung allein.

Beispiel 4

100 ml Faktor VIII Konzentrat, gelöst in 0,15 ml NaCl, wurden mit 0,3 % Tri-n-butylphosphat und 0,2 % Natriumcholat versetzt und 60 min. bei pH 7,2 und 23°C inkubiert. Anschließend wurden 50 μl $\beta$-Propiolacton (0,05 %) zugegeben und die Lösung 90 min. bei pH 7,2 und 23°C inkubiert. Die nachfolgende Tabelle 1 zeigt die Ausbeute an Faktor VIII Aktivität nach $\beta$-Propiolacton-Behandlung nach vorheriger Inkubation mit Tri-n-butylphosphat/Natriumcholat. Die Aktivität von Faktor VIII bleibt nahezu vollständig erhalten.

Beispiel 5

Je 100 ml Plasma oder Faktor VIII-Konzentrat wurden mit dem Bakteriophagen Øx 174 versetzt und sowohl mit als auch ohne Vorbehandlung mit Tri-n-butylphosphat/Natriumcholat (TNBP/NC) mit $\beta$-Propiolacton (0,05 %) sterilisiert. Das Ergebnis zeigt die nachfolgende Tabelle 1.

Der Bakteriophage Øx 174 besitzt keine Lipidhülle und wird erwartungsgemäß durch TNBP/NC nicht inaktiviert. Beobachtet wird jedoch eine deutliche Steigerung der viruziden Wirkung von $\beta$-PL durch die Vorbehandlung mit TNBP/NC.

Tabelle 1

| Inaktivierung von Øx 174 durch TNBP/NC, $\beta$-PL und die Kombination $\beta$-PL/TNBP-NC | | | | |
|---|---|---|---|---|
| Präparat | Behandlung | Inaktivierung Øx 174 ($\log_{10}$) | Steigerung der Effektivität | Ausbeute F VIII (%) |
| Plasma | 0,05 % $\beta$-PL | 1,16 | - | 78 |
| | TNBP-NC* | 0,20 | - | 58 |
| | TNBP-NC* 0,05 % $\beta$-PL | 3,71 | x 355 | 62 |
| | Kontrolle unbehandelt | - | - | 100 |
| Plasma | 0,25 % $\beta$-PL | 2,30 | - | - |
| | TNBP-NC* | 0,02 | - | - |
| | TNBP-NC* 0,25 % $\beta$-PL | 7,16 | x 72440 | - |
| | Kontrolle unbehandelt | - | - | - |
| F VIII | 0,05 % $\beta$-PL | 0,78 | - | 95 |
| | TNBP-NC* | 0,16 | - | 100 |
| | TNBP-NC* 0,05 % $\beta$-PL | 2,04 | x 18 | 93 |
| | Kontrolle unbehandelt | - | - | 100 |

*0,2 % bis 0,3 %

Beispiel 6

Je 100 ml Faktor VIII-Konzentrat wurden mit dem Bakteriophagen Kappa versetzt und sowohl mit als auch ohne Vorbehandlung mit 0,3 % Tris-n-butylphosphat und 1 % Tween 80 mit UV-Licht bestrahlt (2 Röhren 20 W). Die Probe ohne Vorbehandlung mit TNBP/Tween® 80 wurde nach der UV-Bestrahlung mit TNBP-/Tween 80 behandelt. Die Ergebnisse sind in Tabelle 2 dargestellt.

Tabelle 2

| Behandlung | Inaktivierung Kappa ($\log_{10}$) | Ausbeute F VIII (%) |
|---|---|---|
| UV | 3,6 | - |
| UV + TNBP/Tween® 80 | 3,8 | 79 |
| TNBP/Tween® 80 | 0,4 | - |
| TNBP/Tween 80 + UV | 5,4 | 74 |

Die Behandlung mit TNBP/Tween® 80 mit anschließender UV-Bestrahlung (log 5.4) ist somit rund 60 mal wirksamer bei der Inaktivierung des Proteinviren Kappa als die UV-Bestrahlung alleine (log 3.6). TNBP/Tween® 80 allein ist nahezu unwirksam.

**Patentansprüche**

1.  Verfahren zur Sterilisation von Plasma oder Plasmafraktionen einschließlich solcher Fraktionen, die den Blutgerinnungsfaktor VIII enthalten, unter Behandlung mit $\beta$-Propiolacton oder UV-Strahlung, dadurch gekennzeichnet, daß man vor oder gleichzeitig mit der Behandlung mit $\beta$-Propiolacton oder UV-Strahlung eine Behandlung mit Tri-n-butyl-phosphat und Natriumcholat oder Tween® 80 durchführt.

2.  Verfahren nach Anspruch 1, dadurch gekennzeichnet, daß man die Sterilisation bei einem pH-Wert von 4,5 bis 8,3 und einer Temperatur von +4 bis 37°C durchführt.

3.  Verfahren nach einem der Ansprüche 1 bis 2, dadurch gekennzeichnet, daß man das Tri-n-butylphosphat in einer Konzentration von 0,05 bis 0,5 % und das Natriumcholat in einer Konzentration von 0,03 bis 0,4 % anwendet.

4.  Verfahren nach einem der Ansprüche 1 bis 3, dadurch gekennzeichnet, daß man das Tri-n-butylphosphat in einer Konzentration von 0,3 % und das Natriumcholat in einer Konzentration von 0,2 % anwendet.

5.  Verfahren nach einem der Ansprüche 1 bis 3, dadurch gekennzeichnet, daß man das Tri-n-butylphosphat in einer Konzentration von 0,3 % und das Tween® 80 in einer Konzentration von 0,15 - 2 % anwendet.

6.  Verfahren nach einem der Ansprüche 1 bis 5, dadurch gekennzeichnet, daß man $\beta$-Propiolacton in einer Konzentration von 0,01 bis 0,3 % anwendet.

7.  Verfahren nach einem der Ansprüche 1 bis 6, dadurch gekennzeichnet, daß man die Sterilisation bei einem pH-Wert von 7,2 und einer Temperatur von 23°C durchführt.

8.  Verfahren nach einem der Ansprüche 1 bis 7, dadurch gekennzeichnet, daß man zur Sterilisation eines Präparates, das den Blutgerinnungsfaktor VIII enthält, das $\beta$-Propiolacton in einer Konzentration von 0,01 bis 0,25 % anwendet.

9.  Verfahren nach einem der Ansprüche 1 bis 8, dadurch gekennzeichnet, daß man das $\beta$-Propiolacton in einer Konzentration von 0,05 % anwendet.

10. Verfahren nach einem der Ansprüche 1 bis 9, dadurch gekennzeichnet, daß man mit UV-Licht mit vorzugsweise 254 nm bestrahlt.

**Claims**

1.  A process for the sterilization of plasma or plasma fractions including fractions containing the blood clotting factor VIII by treatment with $\beta$-propiolactone or UV radiation, characterised in that a treatment with tri-n-butyl phosphate and sodium cholate or Tween[(R)] 80 is carried out before or simultaneously with the treatment with $\beta$-propiolactone or UV radiation.

2.  A process according to Claim 1, characterised in that sterilisation is carried out at a pH of from 4.5 to 8.3 and a temperature of from 4 to 37°C.

3.  A process according to one of the Claims 1 to 2, characterised in that tri-n-butylphosphate is used at a concentration of from 0.05 to 0.5% and sodium cholate at a concentration of from 0.03 to 0.4%.

4.  A process according to one of the Claims 1 to 3, characterised in that tri-n-butylphosphate is used at a concentration of 0.3% and sodium cholate at a concentration of 0.2%.

5. A process according to one of the Claims 1 to 3, characterised in that tri-n-butylphosphate is used at a concentration of 0.3% and Tween$^{(R)}$ 80 is used at a concentration of from 0.15 to 2%.

6. A process according to one of the Claims 1 to 5, characterised in that β-propiolactone is used at a concentration of from 0.01 to 0.3%.

7. A process according to one of the Claims 1 to 6, characterised in that sterilisation is carried out at a pH of 7.2 and a temperature of 23°C.

8. A process according to one of the Claims 1 to 7, characterised in that β-propiolactone is used at a concentration of from 0.01 to 0.25% for the sterilisation of a preparation containing the blood clotting factor VIII.

9. A process according to one of the Claims 1 to 8, characterised in that β-propiolactone is used at a concentration of 0.05%.

10. A process according to one of the Claims 1 to 9, characterised in that irradiation is carried out with UV light with preferably 254 nm.

**Revendications**

1. Procédé de stérilisation de plasma ou de fractions plasmatiques, y compris les fractions contenant le facteur de coagulation du sang VIII avec traitement par la β-propiolactone ou un rayonnement UV, caractérisé en ce qu'avant ou en même temps que le traitement avec la β-propiolactone ou le rayonnement UV, on effectue un traitement avec du phosphate de tri-n-butyle et du cholate de sodium ou du Tween® 80.

2. Procédé selon la revendication 1, caractérisé en ce qu'on effectue la stérilisation à un pH de 4,5 à 8,3 et à une température de +4 à 37°c.

3. Procédé selon l'une des revendications 1 à 2, caractérisé en ce qu'on utilise le phosphate de tri-n-butyle à une concentration de 0,05 à 0,5% et le cholate de sodium à une concentration de 0,03% à 0,4%.

4. Procédé selon l'une des revendications 1 à 3, caractérisé en ce qu'on utilise le phosphate de tri-n-butyle à une concentration de 0,3% et le cholate de sodium à une concentration de 0,2%.

5. Procédé selon l'une des revendications 1 à 3, caractérisé en ce qu'on utilise le phosphate de tri-n-butyle à une concentration de 0,3% et le Tween® 80 à une concentration de 0,15-2%.

6. Procédé selon l'une des revendications 1 à 5, caractérisé en ce qu'on utilise la β-propiolactone à une concentration de 0,01 à 0,3%.

7. Procédé selon l'une des revendications 1 à 6, caractérisé en ce qu'on effectue la stérilisation à un pH de 7,2 et à une température de 23°C.

8. Procédé selon l'une des revendications 1 à 7, caractérisé en ce qu'on utilise pour la stérilisation d'une préparation qui contient le facteur de coagulation du sang VIII, la β-propiolactone à une concentration de 0,01 à 0,25%.

9. Procédé selon l'une des revendications 1 à 8, caractérisé en ce qu'on utilise la β-propiolactone à une concentration de 0,05%.

10. Procédé selon l'une des revendications 1 à 9, caractérisé en ce qu'on irradie avec une lumière UV de préférence de 254 nm.